# EUROPEAN PATENT SPECIFICATION

(11) **EP 1 896 445 B1**
(45) Date of publication and mention of the grant of the patent: **10.11.2010**
(21) Application number: 06777296.2
(22) Date of filing: 12.06.2006
(51) Int. Cl.: C07D 317/46, A61K 31/5377, A61P 3/04

(54) **( 6-FLU0R0-BENZ0[l, 3] DIOXOLYL) -MORPHOLIN-4-YL-METHANONES AND THEIR USE AS CBl LIGANDS**
(6-FLU0R-BENZ0[l, 3]DIOXOLYL)-MORPHOLIN-4-YL-METHANONE UND DEREN VERWENDUNG ALS CBl-LIGANDEN
( 6-FLUOROBENZO[1,3] DIOXOLYL)-MORPHOLIN-4-YL-MÉTHANONES ET LEUR EMPLOI EN TANT QUE LIGANDS DE CBI

(30) Priority: 22.06.2005 EP 05105516
(43) Date of publication of application: 12.03.2008
(73) Proprietor: F. Hoffmann-La Roche AG, 4070 Basel (CH)
(72) Inventor: FREI, Beat, CH-5105 Auenstein (CH); PLANCHER, Jean-Marc, F-68220 Hagenthal Le Bas (FR); ROEVER, Stephan, 79594 Inzlingen (DE); ZIMMERLI, Daniel, CH-4410 Liestal (CH)
(74) Representative: Klostermeyer-Rauber, Dörte
(86) International application number: PCT/EP2006/063075
(87) International publication number: WO 2006/136502

(56) References cited:
- WO-A-2004/013120

## Description

The present invention is concerned with new stereospecifically 2-disubstituted benzo[1,3]dioxolyl derivatives, their manufacture, pharmaceutical compositions containing them and their use as medicaments. The compounds of the present invention are useful in treating obesity and other disorders.

In particular, the present invention relates to compounds of formula (I): 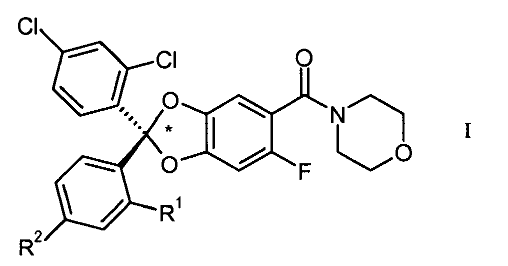 wherein
the asymmetric carbon atom C* is of the R configuration;
R¹ is selected from the group consisting of hydrogen, fluoro or chloro;
R² is hydrogen or chloro,
and pharmaceutically acceptable salts thereof.

Compounds of formula I of the present invention are modulators of the CB1 receptor. They are described generically in PCT patent application No. WO 2004/013120 (Hoffmann-La Roche AG).

Two different subtypes of cannabinoid receptors (CB1 and CB2) have been isolated and both belong to G protein coupled receptor superfamily. Alternative spliced forms of CB 1, CB1A and CB1B have also been described, but are expressed only at low levels in the tissues tested. (D.Shire, C. Carrillon, M. Kaghad, B. Calandra, M. Rinaldi-Carmona, G. Le Fur, D. Caput, P. Ferrara, J. Biol. Chem. 270 (8) (1995) 3726-31; E. Ryberg, H. K. Vu, N. Larsson, T. Groblewski, S. Hjorth, T. Elebring, S. Sjögren, P. J. Greasley, FEBS Lett. 579 (2005) 259-264). The CB1 receptor is mainly located in the brain and to a lesser extent in several peripheral organs, whereas the CB2 receptor is predominately distributed in the periphery primarily localized in spleen and cells of the immune system (S. Munro, K.L. Thomas, M. Abu-Shaar, Nature 365 (1993) 61-61). Therefore in order to avoid side effects a CB1-selective compound is desirable.

Δ⁹-tetrahydrocannabinol (Δ⁹-THC) is the principal psychoactive compound in the Indian hemp (Y. Gaoni, R Mechoulam, J. Am. Chem. Soc., 86 (1964) 1646), *cannabis sativa* (marijuanan), which is used in medicine since ages (R. Mechoulam (Ed.) in "Cannabinoids as therapeutic Agents", 1986, pp. 1-20, CRC Press). Δ⁹-THC is a non-selective CB1/2 receptor agonist and is available in the USA as dronabinol (marinol®) for the alleviation of cancer chemotherapy-induced emesis (CIE) and the reversal of body weight loss experienced by AIDS patients through appetite stimulation. In the UK Nabolinone (LY-109514, Cesamet®), a synthetic analogue of Δ⁹-THC, is used for CIE (R G. Pertwee, Pharmaceut. Sci. 3 (11) (1997) 539-545, E. M. Williamson, F. J. Evans, Drugs 60 (6) (2000) 1303-1314).

Anandamide (arachidonylethanolamide) was identified as the endogenous ligand (agonist) for CB1 (RG. Pertwee, Curr. Med. Chem., 6(8) (1999) 635-664; W.A. Devane, L Hanus, A. Breuer, R.G. Pertwee, L.A. Stevenson, G. Griffin, D. Gibson, A. Mandelbaum, A. Etinger, R Mechoulam, Science 258 (1992) 1946-9). Anandamide and 2-arachidonoylglycerol (2-AG) modulate at the presynaptic nerve terminal negatively adenylate cyclase and voltage-sensitive Ca²⁺ channels and activates the inwardly rectifying K⁺ channel (V. Di Marzo, D. Melck, T. Bisogno, L. De Petrocellis, Trends in Neuroscience 21 (12) (1998) 521-8), thereby affecting neurotransmitter release and/or action, which decreases the release of neurotransmitter (A. C. Porter, C.C. Felder, Pharmacol. Ther., 90 (1) (2001) 45-60).

Anandamide as Δ⁹-THC also increases feeding through CB₁ receptor-mediated mechanism. CB1 selective antagonists block the increase in feeding associated with administration of anandamide (C.M. Williams, T.C. Kirkham, Psychopharmacology 143 (3) (1999) 313-317; C. C. Felder, E. M. Briley, J. Axelrod, J. T. Simpson, K. Mackie, W. A. Devane, Proc. Natl. Acad. Sci. U. S. A. 90(16) (1993) 7656-60) and caused appetite suppression and weight loss (G. Colombo, R. Agabio, G. Diaz, C. Lobina, R. Reali, G. L Gessa, Life Sci. 63 (8) (1998) L113-PL117).

Leptin is the primary signal through which the hypothalamus senses nutritional state and modulates food intake and energy balance. Following temporary food restriction, CB1 receptor knockout mice eat less than their wild-type littermates, and the CB1 antagonist SR141716A reduces food intake in wild-type but not knockout mice. Furthermore, defective leptin signaling is associated with elevated hypothalamic, but not cerebellar, levels of endocannabinoids in obese db/db and ob/ob mice and Zucker rats. Acute leptin treatment of normal rats and ob/ob mice reduces an andamide and 2-arachidonoyl glycerol in the hypothalamus. These findings indicate that endocannabinoids in the hypothalamus may tonically activate CB1 receptors to maintain food intake and form part of the neural circuitry regulated by leptin (V. Di Marzo, S. K. Goparaju, L Wang, J. Liu, S. Bitkai, Z. Jarai, F. Fezza, G. I. Miura, R. D. Palmiter, T. Sugiura, G. Kunos, Nature 410 (6830) 822-825).

At least two CB1 selective antagonist / inverse agonists (SR-141716 and SLV-319) are currently undergoing clinical trials for the treatment of obesity and/or smoking cessation. In a double blind placebo-controlled study, at the doses of 10 and 20 mg daily, SR 141716 significantly reduced body weight when compared to placebo (F. Barth, M. Rinaldi-Carmona, M. Arnone, H. Heshmati, G. Le Fur, "Cannabinoid antagonists: From research tools to potential new drugs." Abstracts of Papers, 222nd ACS National Meeting, Chicago, IL, United States, August 26-30, 2001). SR-141716 reduced body weight, waist circumference and improved metabolic parameters (plasma HDL, triglycerides and insulin sensitivity) in several phase III studies (RIO-lipids, RIO-Europe and RIO-North America). Additionally SR-141716 has shown efficacy in a phase III trial for smoking cessation (STRATUS-US).

Substituted pyrazoles having activity against the cannabinoid receptors are disclosed in US patents 5624941, 6028084 and 6509367, in PCT patent applications WO 98/031227, WO 98/041519, WO 98/043636, WO 98/043635, WO 04/192667, WO 04/0099157 and in patent application EP 658546.

Substituted pyridines, pyrimidines and pyrazines having activity against the cannabinoid receptors are disclosed in US patent application US 04/0259887 and in PCT patent applications WO 03/051850, WO 03/051851, WO 03/084930, WO 04/110453, WO 04/111033, WO 04/111034, WO 04/111038, WO 04/111039 and in patent application FR 2856684.

Other compounds which have been proposed as CB1 receptor antagonists respectively inverse agonists are aminoalkylindols (AAI; M. Pacheco, S. R. Childers, R. Arnold, F. Casiano, S. J. Ward, J. Pharmacol. Exp. Ther. 257 (1) (1991) 170-183). Examples thereof are 6-bromo- (WIN54661; F. M. Casiano, R. Arnold, D. Haycock, J. Kuster, S. J. Ward, NIDA Res. Monogr. 105 (1991) 295-6) or 6-iodopravadoline (AM630, K. Hosohata, R M. Quock, R.M; Hosohata, T. H. Burkey, A Makriyannis, P. Consroe, W. R Roeske, H. I. Yamamura, Life Sci. 61 (1997) 115 - 118; R. Pertwee, G. Griffin, S. Fernando, X. Li, A. Hill, A. Makriyannis, Life Sci. 56 (23-24) (1995) 1949-55). Furthermore, arylbenzo[b]thiophene and benzo[b]furan derivatives (LY320135, C. C. Felder, K. E. Joyce, E. M. Briley, M. Glass, K. P. Mackie, K J. Fahey, G. J. Cullinan, D. C. Hunden, D. W. Johnson, M. O. Chaney, G. A. Koppel, M. Brownstein, J. Pharmacol. Exp. Ther. 284 (1) (1998) 291-7) as disclosed in WO 96/02248 or US5596106, 3-alkyl-(5,5-diphenyl)imidazolidinediones (M. Kanyonyo, S. J. Govaerts, E. Hermans, J. H. Poupaert, D. M. Lambert, Bioorg. Med. Chem. Lett. 9 (15) (1999) 2233 - 2236.) as well as 3-alkyl-5-arylimidazolidinediones (F. Ooms, J. Wouters, O. Oscaro. T. Happaerts, G. Bouchard, P.-A. Carrupt, B. Testa, D. M. Lambert, J. Med. Chem. 45 (9) (2002) 1748-1756) are known to antagonize the CB₁ receptor respectively act as an inverse agonist on the hCB₁ receptor. In WO 00/15609 (FR2783246-A1), WO 01/64634 (FR2805817-A1), WO 02/28346, WO 01/64632 (FR2805818-A1) and WO 01/64633 (FR2805810-A1) are disclosed substituted 1-bis(aryl)methyl-azetidines derivatives as antagonists of CB₁. In WO 01/70700 4,5-dihydro-1H-pyrazole derivatives are described as CB₁ antagonists. In several patent documents bridged and non-bridged 1,5-diphenyl-3-pyrazolecarboxamide derivatives are disclosed as CB₁ antagonists/in verse agonists (WO 01/32663, WO 00/46209, WO 97/19063, EP 658546, EP 656354, US 5624941, EP 576357 and US3940418). However, there still remains a need for potent low molecular weight CB1 modulators that have improved pharmacokinetic and pharmacodynamic properties suitable for use as human pharmaceuticals.

CB1 receptor antagonists respectively inverse agonists having the general formula 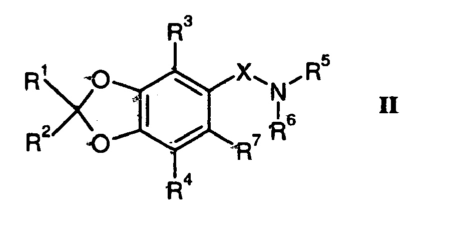 wherein
R¹ and R² are independently unsubstituted phenyl, or phenyl which is mono-, di-or tri-substituted, independently, by hydroxy, lower alkyl, lower alkoxy, perfluoro-lower alkyl, perfluoro-lower alkoxy, alkanoyl, cyano, nitro or halogen; or R¹ and R² together with the carbon atom to which they are attached form a 10',11'-dihydro-2,5'-[5H]dibenzo[a,d]cycloheptene residue;
R³ and R⁴ are independently hydrogen, halogen, hydroxy, lower alkyl, lower alkoxy, perfluoro-lower alkyl, alkanoyl or cyano;
R⁵ is hydrogen, lower alkyl, lower alkylsulfonyl, cycloalkyl lower alkyl or hydroxy-lower alkyl;
R⁶ is Y-R⁸, lower alkyl, lower alkoxy, hydroxy-lower alkyl, lower alkoxy-lower alkyl, lower alkylaminocarbonyl-lower alkyl, heterocyclyl, cycloalkyl, phenyl or phenyl lower alkyl, wherein the phenyl moiety may optionally be mono-, di- or tri-substituted, independently, by lower alkyl, lower alkoxy, halogen, perfluoro-lower alkyl, hydroxy, alkanoyl or cyano; or
R⁶ is hydrogen when X is -C(O)- or -SO₂-; or
R⁵ and R⁶ together with the nitrogen atom to which they are attached form a 4-, 5-, 6- or 7-membered monocyclic or a 8-, 9-, 10-, or 12-membered bicyclic, saturated or unsaturated heterocyclic ring which may optionally contain one or two further heteroatoms independently selected from O, N and S, said heterocyclic ring being optionally mono-, di- or tri-substituted, independently, by lower alkyl, lower alkoxycarbonyl, hydroxy lower alkyl, lower alkoxy-lower alkyl, di-lower alkylcarbamoyl, carbamoyl, lower alkylcarbonyl amino, oxo, dioxo, alkanoyl, amino lower alkyl, hydroxy, lower alkoxy, halogen, perfluoro-lower alkyl, cyano, heteroaryl, or by phenyl or phenyl lower alkyl, wherein the phenyl moiety may optionally be mono-, di- or tri-substituted, independently, by lower alkyl, lower alkoxy, halogen, perfluoro-lower alkyl, hydroxy, alkanoyl or cyano;
R⁷ is hydrogen, halogen, lower alkyl or cyano;
R⁸ is phenyl, cycloalkyl, heterocyclyl or heteroaryl;
X is a single bond, -CH₂- , -C(O)- , -SO₂- or -SO₂NH-;
Y is -CH₂-, -C(O)-, -NH- or -SO₂-;
and pharmaceutically acceptable salts thereof, are described in WO 2004/013120 (Hoffmann-La Roche AG).

It has now been found that the enantiomers of the formula 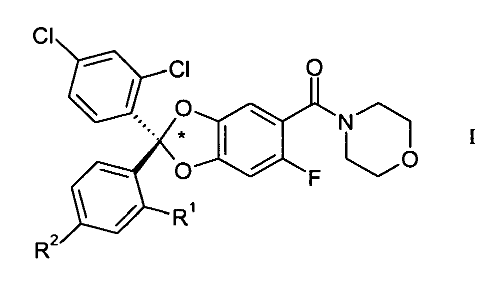 wherein the asymmetric carbon atom C* is of the R configuration;
R¹ is selected from the group consisting of hydrogen, fluoro or chloro;
R² is hydrogen or chloro; and pharmaceutically acceptable salts thereof
are specifically selective towards the CB1 receptor and have therefore advantageous properties.

Unexpectedly it was found that although the enantiomeric compounds described herein may differ only by the position of one halogen atom, *in vivo* properties describing CB1 receptor dependent efficacy may differ substantially. Comparison of the more potent enantiomer with its antipode but also with closely related meso-compounds described in WO 2004/013120 showed unexpected strong advantages for the more potent enantiomers as described below for the hypothermia assay.

*In vivo* efficacy in the hypothermia model translated into efficacy in a disease related model, namely body weight reduction in obese Spague-Dawley rats fed a high fat diet (in analogy to Vickers et al. Psychopharmacology 2003, 167, 103-11 for obese *fa*/*fa* rats). Specifically once daily application of 3 mg/kg po of R-(-)-[2-(4-chloro-phenyl)-2-(2,4-dichloro-phenyl)-6-fluoro-benzo[1,3]dioxol-5-yl]-morpholin-4-yl-methanone led to a BW reduction superior to once daily 10 mg/kg po rimonabant (9.7 vs. 6.6% reduction over 17 days).

Unless otherwise indicated, the following definitions are set forth to illustrate and define the meaning and scope of the various terms used to describe the invention herein.

The term "halogen" refers to fluorine, chlorine, bromine and iodine, preferably to chlorine and fluorine.

The term "pharmaceutically acceptable salts" embraces salts of the compounds of formula (I) with inorganic or organic acids such as hydrochloric acid, hydrobromic acid, nitric acid, sulphuric acid, phosphoric acid, citric acid, formic acid, maleic acid, acetic acid, fumaric acid, succinic acid, tartaric acid, methanesulphonic acid, salicylic acid, p-toluenesulphonic acid and the like, which are non toxic to living organisms. Preferred salts with acids are formates, maleates, citrates, hydrochlorides, hydrobromides and methanesulfonic acid salts, with hydrochlorides being especially preferred.

The term "asymmetric carbon atom" (C*) means a carbon atom with four different substituents. According to the Cahn-Ingold-Prelog-Convention the asymmetric carbon atom can be of the "R" or "S" configuration.

Compounds of the formula 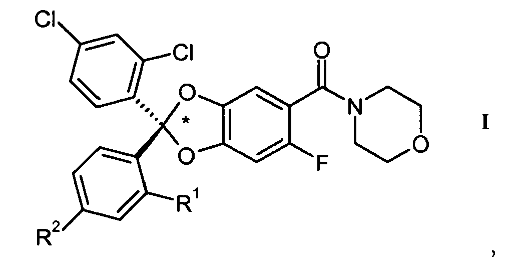 are those, wherein the asymmetric carbon atom C* is of the R configuration.

In one embodiment, the present invention relates to compounds of formula I as defined above, wherein R¹ is fluoro or chloro and R² is hydrogen.

In a preferred embodiment, the present invention relates to a compound of formula I as defined above, wherein R¹ is fluoro and R² is hydrogen.

Thus, (R)-2-(2,4-dichlorophenyl)-2-(2-fluorophenyl)-6-fluoro-benzo[1,3]dioxol-5-yl-morpholin-4-yl-methanone is especially preferred.

In another preferred embodiment, the present invention relates to a compound of formula I as defined above, wherein R¹ is hydrogen and R² is chloro.

Thus, (R)-2-(4-chlorophenyl)-2-(2,4-dichlorophenyl)-6-fluoro-benzo[1,3]dioxol-5-yl-morpholin-4-yl-methanone is especially preferred.

Processes for the manufacture of compounds of formula I are also an object of the invention. Thus, the invention further relates to a process for the manufacture of compounds of formula I, which process comprises:
reacting a compound of the formula 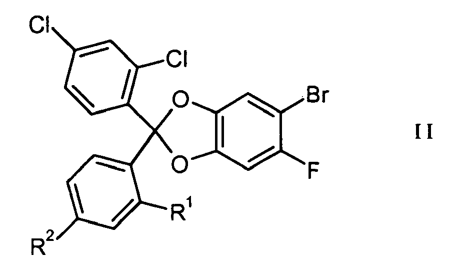 wherein R¹ and R² are as defined herein before in the presence of a strong base with
   4-morpholinecarbonylchloride to obtain a compound of formula 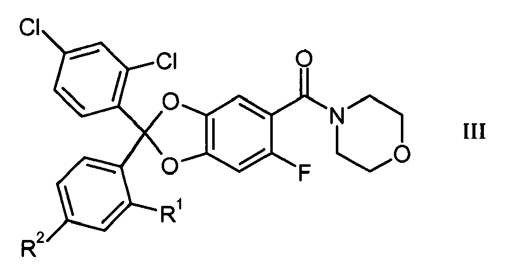 wherein R¹ and R² are as defined herein before;
   and isolating the R-isomer of formula I by preparative chiral HPLC.

More preferably, the invention relates to a process for the manufacture of (R)-2-(2,4-dichlorophenyl)-2-(2-fluorophenyl)-6-fluoro-benzo [1,3]dioxol-5-yl-morpholin-4-yl-methanone, which process comprises:
reacting a compound of the formula 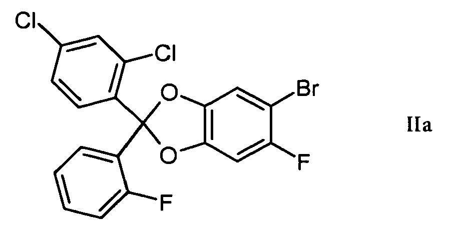
in the presence of a strong base with 4-morpholinecarbonylchloride to obtain a compound of formula 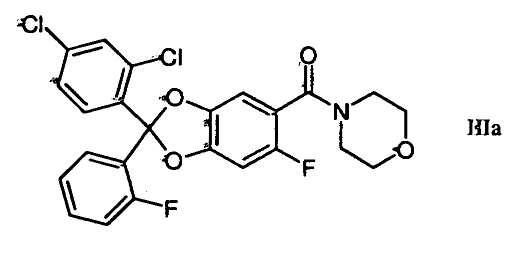 and isolating the R-isomer of formula I by preparative chiral HPLC.

The invention thus also relates to a new intermediate of formula IIIa, namely 2-(2,4-dichlorophenyl)-2-(2-fluorophenyl)-6-fluoro-benzo[1,3]dioxol-5-yl-morpholin-4-yl-methanone.

In detail, the compounds of formula I wherein R¹ and R² are as previously defined may be prepared using the general method depicted in Scheme 1 as further described below or by the methods given in the Examples or by analogous methods. Appropriate reaction conditions for the individual reaction steps are known to the person skilled in the art. Starting materials are either commercially available or can be prepared by methods analogous to the methods given below or in the Examples or by methods known in the art.

According to Scheme 1, a catechol intermediate of formula (B) can be ketalized with a bis-substituted dichloromethane derivative of formula (A) in an inert solvent (e.g. toluene or pyridine) or neat, with or without the presence of a base (e.g. pyridine) at elevated temperature (e.g. > 100 °C) to yield a product of formula (C). A compound of formula (C) can then be coupled with the appropriate acid chloride of formula (D) (4-morpholinecarbonylchloride) in the presence of a strong base such as n-butyl lithium (n-BuLi) in a suitable inert solvent (e.g. diethylether) and reduced temperature (-78 °C) to yield benzodioxoles of formula (III).

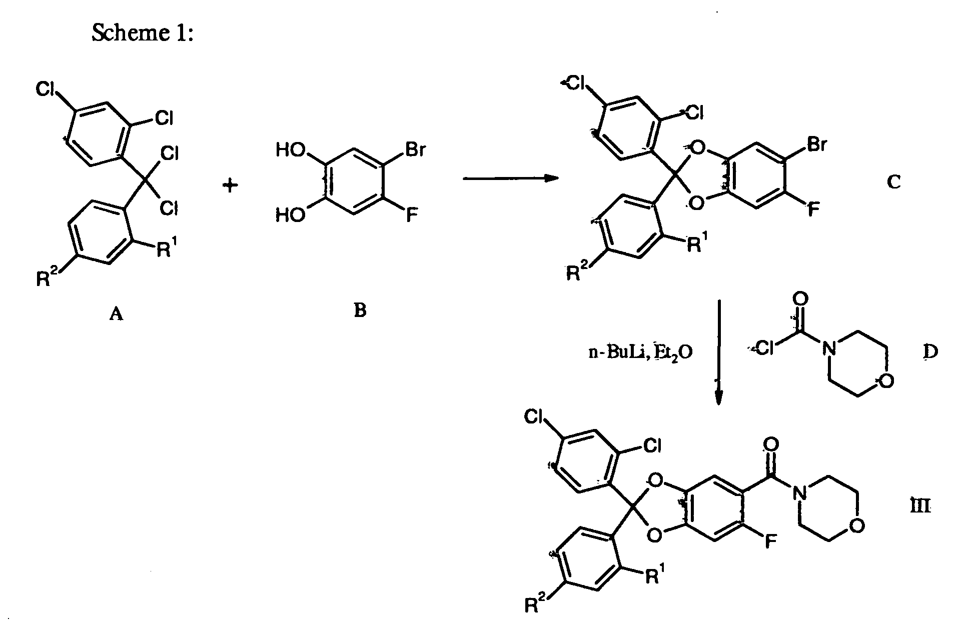

Alternatively, the compound of formula (C) can be converted into the corresponding carboxylic acid of formula (E) by reaction under reduced temperature (-78 °C) with solid carbon dioxide in the presence of a strong base such as n-butyl lithium (n-BuLi) in a suitable inert solvent (e.g. diethylether). The carboxylic acid of formula (E) is then coupled with morpholine after activation with an appropriate coupling agents such as N,N'-carbonyldiimidazole (CDI), N,N'-dicyclohexylcarbodiimide (DCC), 1-(3-dimethylaminopropyl)-3-ethylcarbodiimide hydrochloride (EDCI), 1-[bis(dimethyl-amino)methylene]-1H-1,2,3-triazolo[4,5-b]pyridinium-3-oxide hexafluorophosphate (HATU), 1-hydroxy-1,2,3-benzotriazole (HOBT) or O-benzotriazol-1-yl-N,N,N',N'-tetramethyluronium tetrafluoroborate (TBTU) in a suitable solvent such as dimethylformamide (DMF), dichloromethane, dioxane or tetr-ahydrofuran to yield the amide of formula III (Scheme 2). 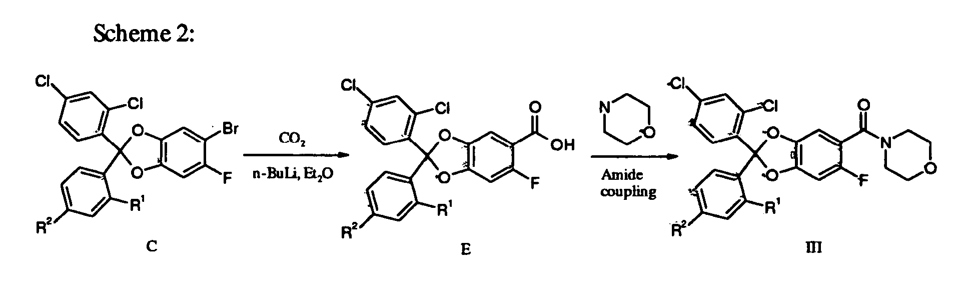 As shown in Scheme 3, the bis substituted dichloromethane derivatives of formula (A) can be easily prepared by methods known in the art from the corresponding ketone of formula (F) by reaction with thionyl chloride in the presence of DMF or another N-formylated agent, by reaction with phosphorus pentachloride at elevated temperature (e.g. > 100 °C) with or without the presence of a suitable solvent (e.g. phosphorus oxide chloride), by electrophilic aromatic substitution of the trifluoromethyl derivative of formula (H) with 1,3-dichlorobenzene (G) in the presence of a Lewis acid (e.g. aluminium trichloride) in an inert solvent (e.g. 1,2-dichloroethane) (e.g. R. K. Ramchandani, R D. Wakharkar, A. Sudalai, Tetrahedron Lett. 37(23) (1996) 4063), or by chlorination of a bisarylmethane derivative of formula (K) (e.g. US 5578737 or W. Deuschel, Helv. Chim. Acta 34 (1951) 2403).

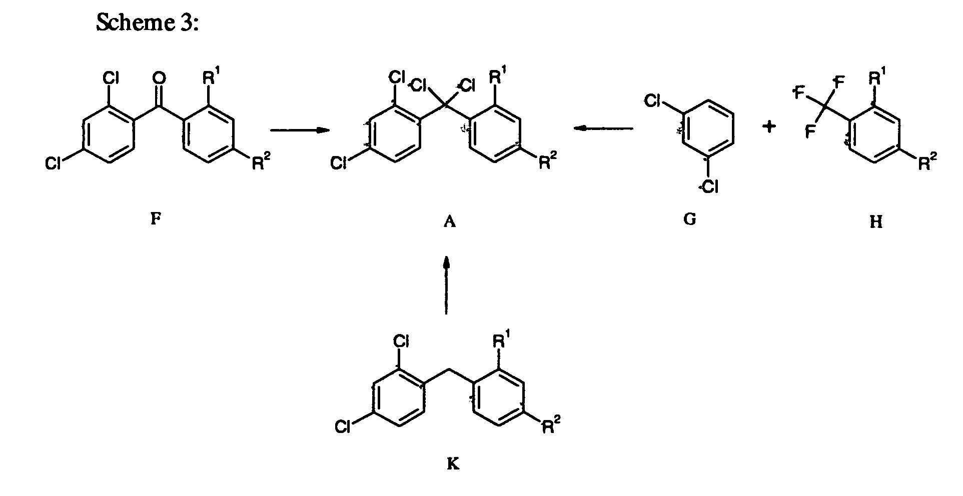

The invention relates to compounds of formula I in substantially pure isomeric form at the asymmetric centre. Compounds of formula III can be separated into optically pure enantiomers by methods well known in the art, e. g. by chromatography on a chiral HPLC column such as ChiralPAK AD or by chromatography with a chiral eluant. Alternatively, the intermediate carboxylic acid (E) can be derivatized with an optically pure alcohol to form diastereomeric esters, which can be separated by conventional HPLC chromatography and then converted back to the enantiomerically pure acids and further coupled to the amide of formula I. In addition, racemic compounds of formula (E) may be separated into their antipodes via diastereomeric salts by crystallization with optically pure alcohols such as (+)- and (-)-menthol.

As described above, the compounds of formula (I) or pharmaceutically acceptable salts thereof can be used as therapeutically active substances, especially as therapeutically active substances for the treatment and/or prophylaxis of diseases which are associated with the modulation of the CB1 receptors.

In one embodiment, the invention therefore relates to compounds as defined above for use as therapeutic active substances, particularly as therapeutic active substances for the treatment and/or prophylaxis of diseases which are associated with the modulation of CB1 receptors.

The invention also relates to pharmaceutical compositions comprising a therapeutically effective amount of a compound as defined above and a pharmaceutically acceptable carrier and/or adjuvant.

In another embodiment, the invention relates to a method for the treatment and/or prophylaxis of diseases which are associated with the modulation of CB1 receptors, which method comprises administering a compound as defined above to a human being or animal.

The invention further relates to the use of compounds as defined above for the treatment and/or prophylaxis of diseases which are associated with the modulation of CB1 receptors.

In addition, the invention relates to the use of compounds as defined above for the preparation of medicaments for the treatment and/or prophylaxis or diseases which are associated with the modulation of CB1 receptors. Such medicaments comprise a compound as defined above.

In this context, the expression 'diseases associated with modulation of CB1 receptors' means diseases which can be treated and/or prevented by modulation of CB1 receptors. Such diseases encompass, but are not limited to, psychic disorders, especially anxiety, psychosis, schizophrenia, depression, abuse of psychotropes, for example for the abuse and/or dependence of a substances, including alcohol dependency and nicotine dependency, neuropathies, multiple sclerosis, migraine, stress,-epilepsy, dyskinesias, Parkinson's disease, amnesia, cognitive disorders, memory deficits, senile dementia, Alzheimer's disease, eating disorders, obesity, diabetes type II or non insulin dependent diabetes (NIDD), gastrointestinal diseases, vomiting, diarrhea, urinary disorders, cardiovascular disorders, infertility disorders, inflammations, infections, cancer, neuroinflammation, in particular in atherosclerosis, or the Guillan-Barré syndrome, viral encephalitis, cerebral vascular incidents and cranial trauma.

In a preferable aspect, the expression 'diseases associated with modulation of CB1 receptors' relates to eating disorders, obesity, diabetes type II or non insulin dependent diabetes (NIDD), neuroinflammation, diarrhea, abuse and/or dependence of a substances, including alcohol dependency and nicotine dependency. In a more preferable aspect, the said term related to eating disorders, obesity, diabetes type II or non insulin dependent diabetes (NIDD), abuse and/or dependence of a substances, including alcohole dependency and nicotine dependency, with obesity being especially preferred.

It is a further preferred object to provide a method for the treatment or prevention of obesity and obesity related disorders which comprises administration of a therapeutically effective amount of a compound according to formula I in combination or association with a therapeutically effective amount of other drugs for the treatment of obesity or eating disorders so that together they give effective relief. Suitable other drugs include but are not limited to anorectic agents, lipase inhibitors and selective serotonin reuptake inhibitors (SSRI). Combinations or associations of the above agents may be encompassing separate, sequential or simultaneous administration.

Preferable lipase inhibitor is tetrahydrolipstatin.

Suitable anorectic agents of use in combination with a compound of the present invention include, but are not limited to, aminorex, amphechloral, amphetamine, benzphetamine, chlorphentermine, clobenzorex, cloforex, clominorex, clortermine, cyclexedrine, dexfenfluramine, dextroamphetamine, diethylpropion, diphemethoxidine, N-ethylamphetamine, fenbutrazate, fenfluramine, fenisorex, fenproporex, fludorex, fluminorex, furfurylmethylamphetamine, levamfetamine, levophacetoperane, mazindol, mefenorex, metamfepramone, methamphetamine, norpseudoephedrine, pentorex, phendimetrazine, phenmetrazine, phentermine, phenylpropanolamine, picilorex and sibutramine, and pharmaceutically acceptable salts thereof.

Most preferable anorectic agents are sibutramine and phentermine.

Suitable selective serotonin reuptake inhibitors of use in combination with a compound of the present invention include: fluoxetine, fluvoxamine, paroxetine and sertraline, and pharmaceutically acceptable salts thereof.

It is a further preferred object to provide a method of treatment or prevention of Type II diabetes (non-insulin dependent diabetes mellitus (NIDDM) in a human which comprises administration of a therapeutically effective amount of a compound according to formula I in combination or association with a therapeutically effective amount of a lipase inhibitor, particularly, wherein the lipase inhibitor is orlistat. Also an object of the invention is the method as described above for the simultaneous, separate or sequential administration of a compound according to formula I and a lipase inhibitor, particularly tetrahydrolipstatin.

It is a further preferred object to provide a method of treatment or prevention of Type II diabetes (non-insulin dependent diabetes mellitus (NIDDM) in a human which comprises administration of a therapeutically effective amount of a compound according to formula I in combination or association with a therapeutically effective amount of an anti-diabetic agent selected from the group consisting of 1) PPARγ agonists such as pioglitazone or rosiglitazone, and the like; 2) biguanides such as metformin, and the like; 3) sulfonylureas such as glibenclamide, and the like; 4) PPARα/γ agonists such as GW-2331, and the like 5) DPP-IV- inhibitors such as LAF-237 (Vildagliptin) or MK-0431, and the like; 6) Glucokin ase activators such as the compounds disclosed in e.g. WO 00/58293 A1, and the like. Also an object of the invention is the method as described above for the simultaneous, separate or sequential administration of a compound according to formula I and a therapeutically effective amount of an anti-diabetic agents as 1) PPARγ agonists such as pioglitazone or rosiglitazone, and the like; 2) biguanides such as metformin, and the like; 3) sulfonylureas such as glibenclamide, and the like; 4) PPARα/γ agonists such as GW-2331 GW-2331 and the like; 5) DPP-IV- inhibitors such as LAF-237 (Vildagliptin) or MK-0431, and the like; 6) Glucokinase activators such as the compounds disclosed in e.g. WO 00/58293 A1, and the like.

It is a further preferred object to provide a method of treatment or prevention of dyslipidemias in a human which comprises administration of a therapeutically effective amount of a compound according to formula I in combination or association with a therapeutically effective amount of a lipid lowering agent as 1) bile acid sequestrants such as cholestyramine, and the like; 2) HMG-CoA reductase inhibitors such as atorvastatin, and the like; 3) cholesterol absorption inhibitors such as ezetimibe, and the like; 4) CETP inhibitors such as torcetrapib, JTT 705, and the like; 5) PPARα-agonists such as beclofibrate, fenofibrate, and the like; 6) lipoprotein synthesis inhibitors such as niacin, and the like; and 7) niacin receptor agonists. Also an object of the invention is the method as described above for the simultaneous, separate or sequential administration of a compound according to formula I and a therapeutically effective amount of a lipid lowering agent as 1) bile acid sequestrants such as cholestyramine, and the like; 2) HMG-CoAreductase inhibitors such as atorvastatin, and the like; 3) cholesterol absorption inhibitors such as ezetimibe, and the like; 4) CETP inhibitors such as torcetrapib, JTT 705, and the like; 5) PPARα-agonists such as beclofibrate, fenofibrate, and the like; 6) lipoprotein synthesis inhibitors such as niacin, and the like; and 7) niacin receptor agonists.

Demonstration of additional biological activities of the compounds of the present invention may be accomplished through in vitro, ex vivo, and in vivo assays that are well known in the art. For example, to demonstrate the efficacy of a pharmaceutical agent for the treatment of obesity-related disorders such as diabetes, Syndrome X, or atherosclerotic disease and related disorders such as hypertriglyceridemia and hypercholesteremia, the following assays may be used.

### Method for Measuring Blood Glucose Levels

db/db mice (obtained from Jackson Laboratories, Bar Harbor, ME) are bled (by either eye or tail vein) and grouped according to equivalent mean blood glucose levels. They are dosed orally (by gavage in a pharmaceutically acceptable vehicle) with the test compound once daily for 7 to 14 days. At this point, the animals are bled again by eye or tail vein and blood glucose levels are determined.

### Method for Measuring Triglyceride Levels

hApoAl mice (obtained from Jackson Laboratories, Bar Harbor, ME) are bled (by either eye or tail vein) and grouped according to equivalent mean serum triglyceride levels. They are dosed orally (by gavage in a pharmaceutically acceptable vehicle) with the test compound once daily for 7 to 14 days. The animals are then bled again by eye or tail vein, and serum triglyceride levels are determined.

### Method for Measuring HDL-Cholesterol Levels

To determine plasma HDL-cholesterol levels, hApoAl mice are bled and grouped with equivalent mean plasma HDL-cholesterol levels. The mice are orally dosed once daily with vehicle or test compound for 7 to 14 days, and then bled on the following day. Plasma is analyzed for HDL-cholesterol.

In addition, to demonstrate CNS activities of the compounds of the present invention, the following in vivo assays may be used.

### Method for Testing Task Learning and Spatial Memory

The Morris Water Maze is routinely used to assess task learning and spatial memory (Jaspers et al., Neurosci. Lett. 117:149-153, 1990; Morris, J. Neurosci. Method 11:47-60, 1984). In this assay, animals are placed in a water pool which is divided into quadrants. One platform is hidden in one of the quadrants. The animal is placed in the water pool and is expected to locate the hidden platform within a predetermined time. During a number of training trials, the animal learns the location of the platform and escapes from the pool. The animal receives multiple trials in this task. Total distance traveled, number of trials to locate platform, latency to find platform, and the swimming path is recorded for each animal. The animal's learning ability is measured by the length of time or number of trials required to find the hidden platform. Memory deficit or improvement is determined by the number of trials or the latency to find the platform at predetermined delay time after acquisition. Leaning and memory may be measured by the number of times that the animal crosses the quadrant where the platform was located during the acquisition phase.

### Method for Testing Drug Dependence

Self-administration in animals is a predictor of a compound's abuse potential in humans. Modifications to this procedure may also be used to identify compounds that prevent or block the reinforcing properties of drugs that have abuse potential. A compound that extinguishes the self-administration of a drug may prevent that drug's abuse or its dependence. (Ranaldi et al., Psychopharmacol. 161:442-448, 2002; Campbell et al., Exp. Clin. Psychopharmacol. 8:312-25, 2000). In a self-administration test, animals are placed in the operant chambers containing both an active and inactive lever. Each response on the active lever produces an infusion of either the test compound or a drug known to be self-administered. Presses on the inactive lever have no effect, but are also recorded. Animals are then trained to self-administer compound/drug over a set period of time by having drug access during each daily session. Illumination of the chamber house light signals the beginning of the session and the availability of the compound/drug. When the session ends, the house light is turned off. Initially, a drug infusion occurs with every press of the active lever. Once lever-pressing behavior has been established, the number of presses to produce a drug infusion is increased. After stable compound/drug self-administration is obtained, the effect of a second compound on the drug-reinforced behavior may be evaluated. Administration of this second compound prior to the session can either potentiate, extinguish, or produce no change to the self-administrating behavior.

The following tests were carried out in order to determine the activity of the compounds of formula (I).

The affinity of the compounds of the invention for cannabinoid CB1 receptors was determined using membrane preparations of human embryonic kidney (HEK) cells in which the human cannabis CB1 receptor is transiently transfected using the Semliki Forest Virus system in conjunction with [3H]-CP-55,940 as radioligand. After incubation of a freshly prepared cell membrane preparation with the [3H]-ligand, with or without addition of compounds of the invention, separation of bound and free ligand was performed by filtration over glassfiber filters. Radioactivity on the filter was measured by liquid scintillation counting.

The affinity of the compounds of the invention for cannabinoid CB2 receptors was determined using membrane preparations of human embryonic kidney (HEK) cells in which the human cannabis CB2 receptor is transiently transfected using the Semliki Forest virus system in conjunction with [3H]-CP-55,940 as radioligand. After incubation of a freshly prepared cell membrane preparation with the [3H]-ligand, with or without addition of compounds of the invention, separation of bound of bound and free ligand was performed by filtration over glassfiber filters. Radioactivity on the filter was measured by liquid scintillation counting.

The cannabinoid CB1 antagonistic activity of compounds of the invention was determined by functional studies using CHO cells in which human cannabinoid CB1 receptors are stably expressed (see M. Rinaldi-Carmona et. al., J. Pharmacol. Exp. Ther. 278 (1996) 871). The stable expression of the human cannabinoid receptor in cell systems was first described in Nature 1990, 346, 561-564 (CB1) and Nature 1993, 365, 61-65 (CB2) respectively. Adenylyl cyclase was stimulated using forskolin and measured by quantifying the amount of accumulated cyclic AMP. Concomitant activation of CB1 receptors by CB1 receptor agonists (e.g. CP-55,940 or (R)-WIN-55212-2) can attenuate the forskolin-induced accumulation of cAMP in a concentration dependent manner. This CB1 receptor mediated response can be antagonised by CB1 receptor antagonists such as the compounds of the invention.

The compounds of formula (I) show an excellent affinity for the CB1 receptor, as can be seen from Table 1 below. The data is determined with the experimental conditions described in Devane et.al. Mol. Pharmacol. 34 (1988) 605-613. The R-isomers or their pharmaceutically acceptable salts are antagonists and selective for the CB1 receptor with affinities below IC₅₀ = 0.01 µM, whereas the S-isomers have affinities over IC₅₀ = 0.01 µM They exhibit at least a 100 fold selectivity against the CB2 receptor.

**Table 1**

| **Compound** | **IC₅₀ [µM]** |
|---|---|
| R-(+)-[2-(2,4-Dichloro-phenyl)-6-fluoro-2-(2-fluoro-phenyl)-benzo[1,3]dioxol-5-yl]-morpholin-4-yl-methanone | 0.005 |
| S-(-)-[2-(2,4-Dichloro-phenyl)-6-fluoro-2-(2-fluoro-phenyl)-benzo[1,3]dioxol-5-yl]-morpholin-4-yl-methanone | 0.033 |
| R-(-)-[2-(4-Chloro-phenyl)-2-(2,4-dichloro-phenyl)-6-fluoro-benzo[1,3]dioxol-5-yl]-morpholin-4-yl-methanone | 0.0025 |
| S-(+)-[2-(4-Chloro-phenyl)-2-(2,4-dichloro-phenyl)-6-fluoro-benzo[1,3]dioxol-5-yl]-morpholin-4-yl-methanone | 0.017 |

### Effect of CB1 receptor antagonist/inverse agonist on CP 55,940-induced Hypothermia in NMRI mice

### Animals

Male NMRI mice were used in this study and were obtained from Research Consulting Company Ltd (RCC) of Füllinsdorf (Switzerland). Mice, weighing 30-31g were used in this study. Ambient temperature is approximately 20-21°C and relative humidity 55-65%. A 12 hours light-dark cycle is maintained in the rooms with all tests being performed during the light phase. Access to tap water and food are ad libitum.

### Method

All measurements were made between 12:00 am and 5:00 pm. Mice were brought in this environment and habituated for at least two hours before the start of the experiment. They had always free access to food and water. For each dose, 8 mice were used. Rectal body temperature measurements were recorded by mean of a rectal probe (RET2 of Physitemp) and digital thermometer (Digi-sense n°8528-20 of Cole Parmer, Chicago USA). The probe was inserted about 3.5 cm in each mouse.

The body temperature was taken 15 min before administration of either Vehicle or CB1 receptor antagonist/inverse agonist. 30 or 90 min after i.p. or p.o. administration of this compound, respectively, rectal body temperature was recorded in order to devaluate any influence of the compound itself. The CB receptor agonist CP 55,940 (0.3 mg/kg) was immediately administered intravenously, then 20 min after i.v. administration of CP 55940, body temperature was again measured.

The ID₅₀ values or minimum effective doses of the compounds of the present invention and of their enantiomers are shown in table 2 below.

**Table 2**

| Compound | CB1 receptor affinity IC₅₀ [µM] | Hypothermia in NMRI mice ID₅₀ or minimum effective dose (MED) [mg/kg po] |
|---|---|---|
| R-(+)-[2-(2,4-Dichloro-phenyl)-6-fluoro-2-(2-fluoro-phenyl)-benzo[1,3]dioxol-5-yl]-morpholin-4-yl-methanone | 0.005 | ID₅₀:40 |
| S-(-)-[2-(2,4-Dichloro-phenyl)-6-fluoro-2-(2-fluoro-phenyl)-benzo[1,3]dioxol-3-yl]-morpholin-4-yl-methanone | 0.033 | inactive at 30 |
| R-(-)-[2-(4-Chloro-phenyl)-2-(2,4-dichloro-phenyl)-6-fluoro-benzo[1,3]dioxol-5-yl]-morpholin-4-yl-methanone | 0.0025 | ID₅₀: 1.6 |
| S-(+)-[2-(4-Chloro-phenyl)-2-(2,4-dichloro-phenyl)-6-fluoro-benzo[1,3]dioxol-5-yl]-morpholin-4-yl-methanone | 0.017 | MED:10 |
| Meso-[2,2-Bis-(4-chloro-phenyl)-6-fluoro-benzo[1,3]dioxol-5-yl]-morpholin-4-yl-methanone | 0.026 | inactive at 30 |

The in vivo activity of compounds of formula (I) was assessed for their ability to regulate feeding behaviour by recording food con sumption in food deprived animals.

Rats were trained to have access to food for 2h per day and were food deprived for 22h. When they were trained under this schedule, the amount of food taken every day during these 2h food intake session was consistent day after day.

To test the ability of compounds of formula (I) to decrease food intake, 8 animals were used in a cross-over study. Rats were individually housed in Plexiglas boxes with a grid on the floor and a paper was placed below the cage floor to collect any spillage. A food dispenser (beaker) filled with a pre-weighed amount of food was presented to them for 2h. At the end of the food intake session, rats returned to their home cage. Each rat was weighed before the start of the experiment and the amount of food consumed during this 2h food intake session was recorded. Either various doses of test compound or vehicle was administered orally 60 min before the 2h food intake session. A positive control Rimonabant (SR141716) was included in the experiment. An Anova analysis with repeated measures was used followed by a posthoc test Student Neumann-Keuls. * P < 0.05 compared to Saline-treated rats.

Furthermore the utility of compounds of formula (I) in diseases or disorders may be demonstrated in animal disease models that have been reported in the literature. The following are examples of such animal disease models: a) reduction of sweet food intake in marmosets (Behavioural Pharm, 1998, 9,179-181); b) reduction of sucrose and ethanol intake in mice (Psychopharm. 1997, 132, 104-106); c) increased motor activity and place conditioning in rats (Psychopharm. 1998, 135, 324-332; Psychopharmacol 2000,151: 25-30); d) spontaneous locomotor activity in mice (J. Pharm. Exp. Ther. 1996, 277, 586-594); e) reduction in opiate self administration in mice (Sci. 1999, 283, 401-404);

The compounds of formula (I) and/or their pharmaceutically acceptable salts can be used as medicaments, e.g. in the form of pharmaceutical preparations for enteral, parenteral or topical administration. They can be administered, for example, perorally, e.g. in the form of tablets, coated tablets, dragées, hard and soft gelatine capsules, solutions, emulsions or suspensions, rectally, e.g. in the form of suppositories, parenterally, e.g. in the form of injection solutions or infusion solutions, or topically, e.g. in the form of ointments, creams or oils. Oral administration is preferred.

The production of the pharmaceutical preparations can be effected in a manner which will be familiar to any person skilled in the art by bringing the described compounds of formula (I) and/or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

Suitable carrier materials are not only inorganic carrier materials, tut also organic carrier materials. Thus, for example, lactose, corn starch or derivatives thereof, talc, stearic acid or its salts can be used as carrier materials for tablets, coated tablets, dragées and hard gelatine capsules. Suitable carrier materials for soft gelatine capsules are, for example, vegetable oils, waxes, fats and semi-solid and liquid polyols (depending on the nature of the active ingredient no carriers might, however, be requited in the case of soft gelatine capsules). Suitable carrier materials for the production of solutions and syrups are, for example, water, polyols, sucrose, invert sugar and the like. Suitable carrier materials for injection solutions are, for example, water, alcohols, polyols, glycerol and vegetable oils. Suitable carrier materials for suppositories are, for example, natural or hardened oils, waxes, fats and semi-liquid or liquid polyols. Suitable carrier materials for topical preparations are glycerides, semi-synthetic and synthetic glycerides, hydrogen ated oils, liquid waxes, liquid paraffins, liquid fatty alcohols, sterols, polyethylene glycols and cellulose derivatives.

Usual stabilizers, preservatives, wetting and emulsifying agents, consistency-improving agents, flavour-improving agents, salts for varying the osmotic pressure, buffer substances, solubilizers, colorants and masking agents and antioxidants come into consideration as pharmaceutical adjuvants.

The dosage of the compounds of formula (I) can vary within wide limits depending on the disease to be controlled, the age and the individual condition of the patient and the mode of administration, and will, of course, be fitted to the individual requirements in each particular case. For adult patients a daily dosage of about 1 to 1000 mg, especially about 1 to 100 mg, comes into consideration. Depending on severity of the disease and the precise pharmacokinetic profile the compound could be administered with one or several daily dosage units, e.g. in 1 to 3 dosage units.

The pharmaceutical preparations conveniently contain about 1-500 mg, preferably 1-100 mg, of a compound of formula (I).

The following Examples serve to illustrate the present invention in more detail. They are, however, not intended to limit its scope in any manner.

### Examples

MS = mass spectrometry, EI = electron impact, ISP = ion spray(positive ion). All experiments were conducted under an inert atmosphere (nitrogen or argon).

### Example 1

### R-(+)-[2-(2,4-Dichloro-phenyl)-6-fluoro-2-(2-fluoro-phenyl)-benzo[1,3]dioxol-5-yl]-morpholin-4-yl-meth an one

### a) Preparation of 4-bromo-5-fluoro-benzene-1,2-diol

To a cooled (-78 °C) solution of 4-fluoroveratrole (5.0 g, 32 mmol) in dichloromethane (106 mL) was slowly added a solution of tribromoborane in dichloromethane (1M, 96 mL, 96 mmol, 3.0 eq.). The reaction mixture was warmed to 20 °C and stirred overnight. The reaction mixture was poured into ice water, extracted with ethyl acetate (3 times). The combined organic layer was washed with an aqueous solution of sodium bicarbonate, dried over sodium sulfate and filtered. The volatiles were removed *in vacuo.* The brown solid was diluted with chloroform (50 mL) and dichloromethane (10 mL). A solution of bromine in carbon tetrachloride (5 ml) was slowly added. After 3 hours, the volatiles were removed *in vacuo.* Purification by flash chromatography afforded the title compound (6.51 g, 98%) as a brown solid
MS: m/e= 207.9 ([M+H]⁺).

### b) Preparation of 2,4-dichloro-2'-fluoro-diphenyldichloromethane

To a cooled (0 °C) suspension of aluminium trichloride (46.4 g, 348 mmol, 3.0 eq.) in 1,2-dichloroethane (160 mL) was slowly added 2-fluorobenzotrifluoride (19.0 g, 116 mmol) followed by 1,3-dichlorobenzene (17.1 g, 116 mmol, 1.0 eq.). The reaction mixture was stirred at 0-5 °C for 3h, then poured onto ice and extracted with dichloromethane. The combined organic layer was washed with brine, dried over sodium sulfate and filtered. The volatiles were removed *in vacuo,* affording the title compound (39.9 g, quant.) as yellow oil.
MS: m/e = 287.0 ([M-Cl*]+).

### c) Preparation of 5-Bromo-2-(2,4-dichloro-phenyl)-6-fluoro-2-(2-fluoro-phenyl)-benzo[1,3]dioxole

A mixture of 4-bromo-5-fluoro-benzene-1,2-diol (15.0 g, 72.5 mmol, 1.2 eq.) and 2,4-dichloro-2'-fluoro-chlorodiphenyldichloromethane (19.6 g, 60.5 mmol, 1.0 eq.) was heated under stirring at 180 °C for 20 min. The reaction mixture was cooled to 20 °c, diluted with dichloromethane and adsorbed onto silica. Purification by flash chromatography afforded the title compound (20.8 g, 75.1 %) as a white amorphous solid.
MS: m/e = 459.0 ([M+H]⁺).

### d) Preparation of [2-(2,4-Dichloro-phenyl)-6-fluoro-2-(2-fluoro-phenyl)-benzo[1,3]dioxol-5-yl]-momholin-4-yl-methanone

To a cooled (-78 °C) solution of 5-bromo-2-(2,4-dichloro-phenyl)-6-fluoro-2-(2-fluoro-phenyl)-benzo[1,3]dioxole(7.0g, 15.3 mmol) in diethylether (100 mL) was slowly added a solution of n-butyl lithium in hexanes (1.6M, 9.55 mL, 15.3 mmol, 1.0 eq.). The reaction mixture was stirred 1h at -78 °C before the addition of 4-morphline-carbonylchloride (3.43 g, 22.9 mmol, 1.5 eq.). The reaction mixture was allowed to warm to 20 °C and poured into an aqueous solution of sodium bicarbonate. The aqueous layer was extracted with ethyl acetate. The combined organic layers were washed with brine. Volatiles were removed *in vacuo.* Purification by flash chromatography afforded the title compound (5.49 g, 73%) as an amorphous white solid.
MS: m/e = 493.3 ([M+H]⁺).

### e) Preparation of R-(+)-[2-(2,4-dichloro-phenyl)-6-fluoro-2-(2-fluoro-phenyl)-benzo[1,3]dioxol-5-yl]-morpholin-4-yl-methanone

The title compound was isolated by preparative chiral HPLC (Analytical HPLC: ChiralPak AD 0.46 x 25 cm; flow: 1 mL.min⁻¹; solvents: heptane/isopropanol 85:15) from racemic [2-(2,4-dichloro-phenyl)-6-fluoro-2-(4-fluoro-phenyl)-benzo[1,3]dioxol-5-yl]-morpholin-4- yl-methanone (Example 1d). White solid.
MS: m/e =493.3 ([M+H]⁺).
Specific rotation : +7.39 °.mLg⁻¹.dm⁻¹ at 589 nm.
Retention time: 17.8 min

### Undesired enantiomer S-(-)-[2-(2,4-dichloro-phenyl)-6-fluoro-2-(2-fluoro-phenyl)-benzo[1,3]dioxol-5-yl]-morpholin-4-yl-methanone:

MS: m/e = 493.3 ([M+H]⁺)
Specific rotation: -7.46°.mLg⁻¹.dm⁻¹ at 589 nm.
Retention time: 12.6 min.

### Example 2

### R-(-)-[2-(4-Chloro-phenyl)-2-(2,4-dichloro-phenyl)-6-fluoro-benzo[1,3]dioxol-5-yl]-morpholin-4-yl-methanone

### a) Preparation of 2,4-dichloro-4'-chloro-diphenyldichloromethane

The title compound was produced in accordance with the general method of Example 1b from 2,4-dichlorobenzotrifluoride and chlorobenzene. Brown oil.
MS: m/e = 338 ([M]⁺).

### b) Preparation of 5-Bromo-2-(4-chloro-phenyl)-2-(2,4-dichloro-phenyl)-6-fluoro-benzo[1,3]dioxole

The title compound was produced in accordance with the general method of Example 1c from 2,4-dichloro-4'-chloro-diphenyldichloromethane and 4-btomo-5-fluoro-benzene-1,2-diol. White amorphous solid.
MS: m/e = 473.9 ([M]⁺).

### c) Preparation of [2-(4-Chloro-phenyl)-2-(2,4-dichloro-phenyl)-6-fluoro-benzo[1,3]dioxol-5-yl]-morpholin-4-yl-methanone

The title compound was produced in accordance with the general method of Example 1d from 5-bromo-2-(4-chloro-phenyl)-2-(2,4-dichloro-phenyl)-6-fluoro-benzo[1,3]dioxole. White amorphous solid.
MS: m/e = 508.3 ([M)⁺).

### d) Preparation of R-(-)-[2-(4-Chloro-phenyl)-2-(2,4-dichloro-phenyl)-6-fluoro-benzo[1,3]dioxol-5-yl]-morpholin-4-yl-methanone

The title compound was isolated by preparative chiral HPLC (Analytical HPLC: ChiralPak AD 0.46 x 25 cm; flow: 1 mL.min⁻¹; solvents: heptane/isopropanol 80:20) from racemic [2-(4-Chloro-phenyl)-2-(2,4-dichloro-phenyl)-b-fluoro-benzo[1,3]dioxol-5-yl]-morpholin-4-yl-methanone (Example 2c). White solid.
MS: m/e = 508.3 ([M]⁺)
Specific rotation : -5.94 °.mL.g⁻¹.dm⁻¹ at 589 nm.
Retention time: 20.5 min

### Undesired enantiomer S-(+)-[2-(4-Chloro-phenyl)-2-(2,4-dichloro-phenyl)-6-fluoro-benzo[1,3]dioxol-5-yl]-morpholin-4-yl-methanone:

MS: m/e = 508.3 ([M]⁺)
Specific rotation : +5.45°.mL.g⁻¹.dm⁻¹ at 589 nm.
Retention time: 25.6 min.

The absolute configuration of the (R)-enantiomer was determined by independent synthesis from (S)-5-bromo-2-(4-chloro-phenyl)-2-(2,4-dichloro-phenyl)-6-fluoro-benzo[1,3]dioxole in accordance with the general method of Example ld. (S)-5-Bromo-2-(4-chloro-phenyl)-2-(2,4-dichloro-phenyl)-6-fluoro-benzo[1,3]dioxole was isolated by preparative chiral HPLC (analytical HPLC: ChiralPak AD 0.46 x 25 cm; flow: 1 mL.min⁻¹; solvents: heptane/isopropanol 98:2; Specific rotation: +3.32 °.mL.g-1.dm⁻¹ at 589 nm; retention time: 6.7 min; (R)-enantiomer had specific rotation: -3.44 °.mL.g-1.dm⁻¹ at 589 nm; retention time: 7.8 min) from racemic 5-bromo-2-(4-chloro-phenyl)-2-(2,4-dichlorophenyl)-6-fluoro-benzo[1,3]dioxole (Example 2b) and its absolute configuration unequivocally determined by X-ray crystallography.

### Galenical Examples

### Example A

Film coated tablets containing the following ingredients can be manufactured in a conventional manner:

| Ingredients | Per tablet | |
|---|---|---|
| **Kernel:** | | |
| Compound of formula (I) | 10.0 mg | 200.0 mg |
| Microcrystalline cellulose | 23.5 mg | 43.5 mg |
| Lactose hydrous | 60.0 mg | 70.0 mg |
| Povidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |
| **Film Coat:** | | |
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxide (yellow) | 0.8 mg | 1.6 mg |
| Titanium dioxide | 0.8 mg | 1.6 mg |

The active ingredient is sieved and mixed with microcrystalline cellulose and the mixture is granulated with a solution of polyvinylpyrrolidone in water. The granulate is mixed with sodium starch glycolate and magnesium stearate and compressed to yield kernels of 120 or 350 mg respectively. The kernels are lacquered with an aq. solution / suspension of the above mentioned film coat.

### Example B

Capsules containing the following ingredients can be manufactured in a conventional manner:

| Ingredients | Per capsule |
|---|---|
| Compound of formula (I) | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of formula (I) | 3.0 mg |
| Polyethylene glycol 400 | 150.0 mg |
| Acetic acid | q.s. ad pH 5.0 |
| Water for injection solutions | ad 1.0 ml |

The active ingredient is dissolved in a mixture of Polyethylene glycol 400 and water for injection (part). The pH is adjusted to 5.0 by addition of acetic acid. The volume is adjusted to 1.0 ml by addition of the residual amount of water. The solution is filtered, filled into vials using an appropriate overage and sterilized.

## Claims

1. Compounds of formula (I) 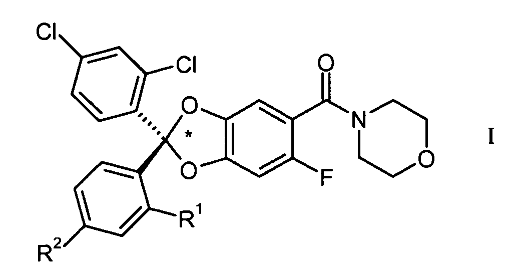 wherein
the asymmetric carbon atom C* is of the R configuration;
R¹ is fluoro or chloro and R² is hydrogen or
wherein R¹ is hydrogen and R² is chloro;
and pharmaceutically acceptable salts thereof.

2. A compound according to claim 1, wherein R¹ is fluoro and R² is hydrogen.

3. A compound according to claim 2, which is (R)-2-(2,4-dichlorophenyl)-2-(2-fluorophenyl)-6-fluoro-benzo[1,3]dioxol-5-yl-morpholin-4-yl-methanone, or a pharmaceutically acceptable salt thereof.

4. A compound according to claim 1, wherein R¹ is hydrogen and R² is chloro.

5. A compound according to claim 4, which is (R)-2-(4-chlorophenyl)-2-(2,4-dichlorophenyl)-6-fluoro-benzo[1,3]dioxol-5-yl-morpholin-4-yl-methanone, or a pharmaceutically acceptable salt thereof.

6. A process for the manufacture of compounds of formula I as defined in any of claims 1 to 5, which process comprises:
reacting a compound of the formula 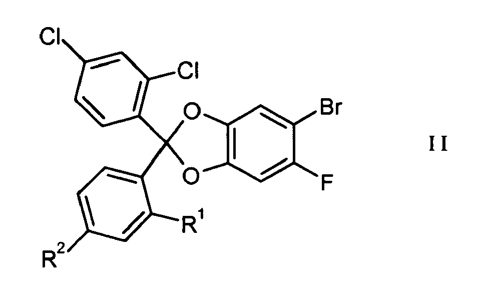
wherein R¹ and R² are as defined in claim 1, in the presence of a strong base with 4-morpholinecarbonylchloride to obtain a compound of formula 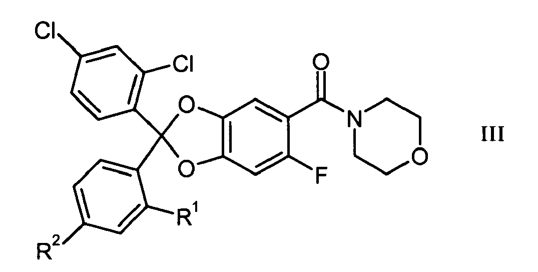 wherein R¹ and R² are as defined in claim 1;
and isolating the R-isomer of formula I by preparative chiral HPLC.

7. A process according to claim 6 for the manufacture of (R)-2-(2,4-dichlorophenyl)-2-(2-fluorophenyl)-6-fluoro-benzo[1,3]dioxol-5-yl-morpholin-4-yl-methanone, which process comprises:
reacting a compound of the formula 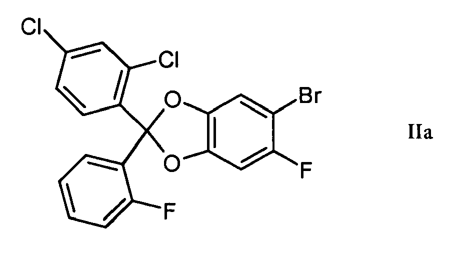 in the presence of a strong base with 4-morpholinecarbonylchloride to obtain a compound of formula 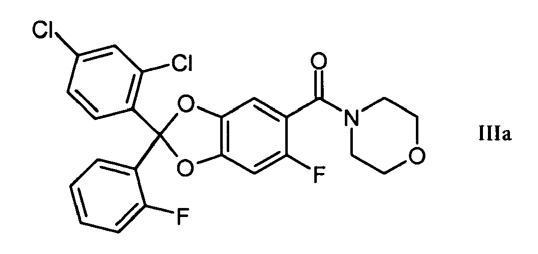 and isolating the R-isomer of formula I by preparative chiral HPLC.

8. The compound of formula **IIIa,** which is 2-(2,4-dichlorophenyl)-2-(2-fluorophenyl)-6-fluoro-benzo [1,3]dioxol-5-yl-morpholin-4-yl-methanone.

9. Pharmaceutical compositions comprising a compound according to any of claims 1 to 5 and a pharmaceutically acceptable carrier and/or adjuvant.

10. Compounds according to any of claims 1 to 5 for use as therapeutic active substances.

11. Compounds according to any of claims 1 to 5 for use as therapeutic active substances for the treatment and/or prophylaxis of diseases which are associated with modulation of the CB1 receptor.

12. The use of compounds according to any of claims 1 to 5 for the preparation of medicaments for the treatment and/or prophylaxis of diseases which are associated with the modulation of CB1 receptors.

## Patentansprüche

1. Verbindungen der Formel (I) 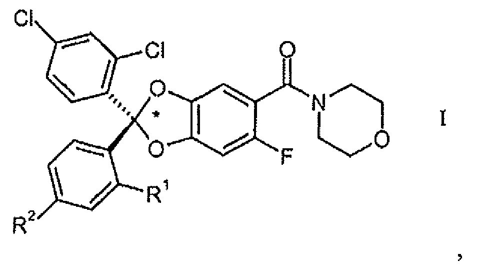 worin
das asymmetrische Kohlenstoffatom C* die R-Konfiguration aufweist;
R¹ Fluor oder Chlor ist und R² Wasserstoff ist oder
worin R¹ Wasserstoff ist und R² Chlor ist;
und pharmazeutisch akzeptable Salze davon.

2. Verbindung nach Anspruch 1, wobei R¹ Fluor ist und R² Wasserstoff ist.

3. Verbindung nach Anspruch 2, die (R)-2-(2,4-Dichlorphenyl)-2-(2-fluorphenyl)-6-fluor-benzo[1,3]dioxol-5-yl-morpholin-4-yl-methanon oder ein pharmazeutisch akzeptables Salz davon ist.

4. Verbindung nach Anspruch 1, wobei R¹ Wasserstoff ist und R² Chlor ist.

5. Verbindung nach Anspruch 4, die (R)-2-(4-Chlorphenyl)-2-(2,4-dichlorphenyl)-6-fluor-benzo[1,3]dioxol-5-yl-morpholin-4-yl-methanon oder ein pharmazeutisch akzeptables Salz davon ist.

6. Verfahren zur Herstellung von Verbindungen der Formel I, wie in einem der Ansprüche 1 bis 5 definiert, wobei das Verfahren:
das Umsetzen einer Verbindung der Formel 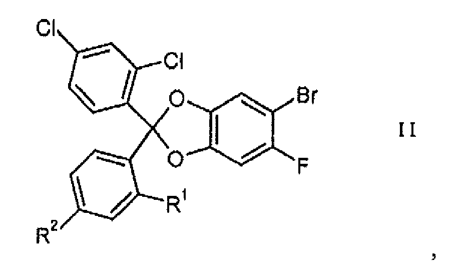 worin R¹ und R² wie in Anspruch 1 definiert sind, in Gegenwart einer starken Base mit 4-Morpholincarbonylchlorid unter Erhalt einer Verbindung der Formel 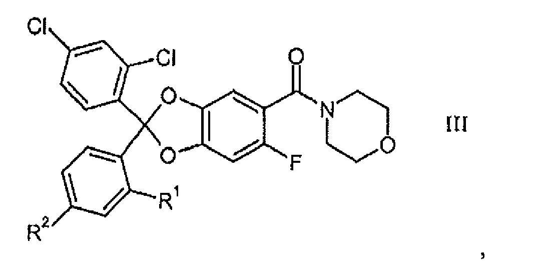 worin R¹ und R² wie in Anspruch 1 definiert sind; und Isolieren des R-Isomers von Formel I durch präparative chirale HPLC umfasst.

7. Verfahren nach Anspruch 6 zur Herstellung von (R)-2-(2,4-Dichlorphenyl)-2-(2-fluor-phcnyl)-6-fluor-bcnzo[1,3]dioxol-5-yl-morpholin-4-yl-mcthanon, wobei das Verfahren:
das Umsetzen einer Verbindung der Formel 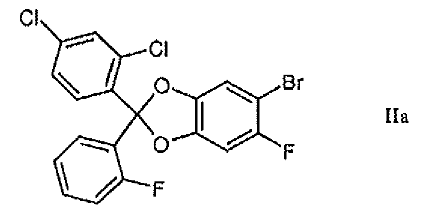 in Gegenwart einer starken Base mit 4-Morpholincarbonylchlorid unter Erhalt einer Verbindung der Formel 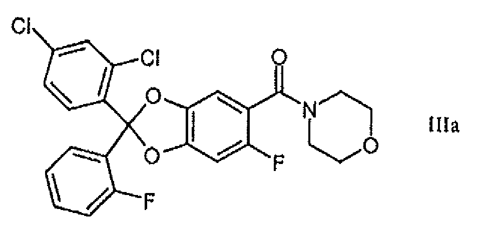 und Isolieren des R-Isomers der Formel I durch präparative chiral HPLC umfasst.

8. Verbindung der Formel IIIa, die 2-(2,4-Dichlorphenyl)-2-(2-fluorphenyl)-6-fluor-benzo[1,3]dioxol-5-yl-morpholin-4-yl-methanon ist.

9. Pharmazeutische Zusammensetzungen, umfassend eine Verbindung nach einem der Ansprüche 1 bis 5 und einen pharmazeutisch akzeptablen Träger und/oder ein pharmazeutisch akzeptables Adjuvans.

10. Verbindungen nach einem der Ansprüche 1 bis 5 zur Verwendung als therapeutisch aktive Substanzen.

11. Verbindungen nach einem der Ansprüche 1 bis 5 zur Verwendung als therapeutisch aktive Substanzen zur Behandlung und/oder Prophylaxe von Krankheiten, die mit der Modulierung des CB1-Rezeptors in Verbindung stehen.

12. Verwendung von Verbindungen nach einem der Ansprüche 1 bis 5 zur Herstellung von Medikamenten zur Behandlung und/oder Prophylaxe von Krankheiten, die mit der Modulierung von CB 1-Rezeptoren in Verbindung stehen.

## Revendications

1. Composés de formule (I) 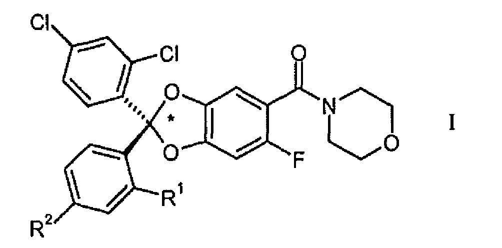 dans laquelle
l'atome de carbone asymétrique C* est sous la configuration R ;
R¹ représente un groupe fluoro ou chloro et R² représente un atome d'hydrogène, ou bien
R¹ représente un atome d'hydrogène et R² représente un groupe chloro ;
et leurs sels pharmaceutiquement acceptables.

2. Composé suivant la revendication 1, dans lequel R¹ représente un groupe fluoro et R² représente un atome d'hydrogène.

3. Composé suivant la revendication 2, qui est la (R)-2-(2,4-dichlorophényl)-2-(2-fluorophényl)-6-fluoro-benzo-[1,3]dioxole-5-yl-morpholine-4-yl-méthanone, ou un de ses sels pharmaceutiquement acceptables.

4. Composé suivant la revendication 1, dans lequel R¹ représente un atome d'hydrogène et R² représente un groupe chloro.

5. Composé suivant la revendication 4, qui est la (R)-2-(4-chlorophényl)-2-(2,4-dichlorophényl)-6-fluoro-benzo-[1,3]dioxole-5-yl-morpholine-4-yl-méthanone, ou un de ses sels pharmaceutiquement acceptables.

6. Procédé pour la production de composés de formule I tels que définis dans l'une quelconque des revendications 1 à 5, procédé qui comprend :
la réaction d'un composé de formule 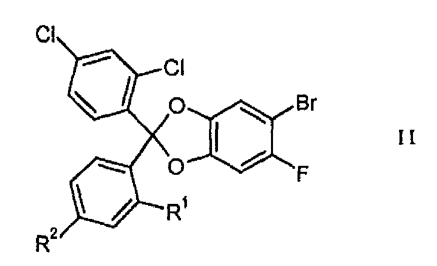 dans laquelle R¹ et R² sont tels que définis dans la revendication 1, en présence d'une base forte, avec du chlorure de 4-morpholinecarbonyle pour obtenir un composé de formule 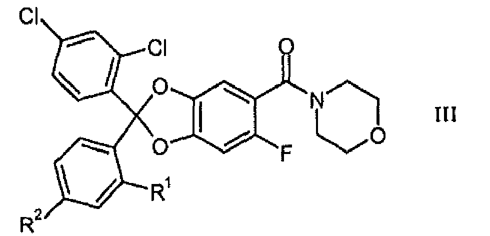 dans laquelle R¹ et R² sont tels que définis dans la revendication 1 ;
et l'isolement de l'isomère R de formule I par HPLC chirale préparative.

7. Procédé suivant la revendication 6, pour la production de (R)-2-(2,4-dichlorophényl)-2-(2-fluorophényl)-6-fluoro-benzo[1,3]dioxole-5-yl-morpholine-4-yl-méthanone, procédé qui comprend :
la réaction d'un composé de formule 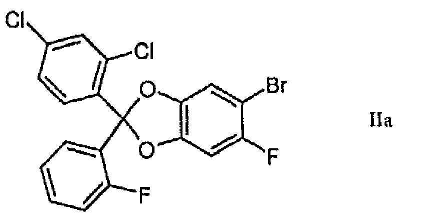
en présence d'une base forte, avec du chlorure de 4-morpholinecarbonyle pour obtenir un composé de formule 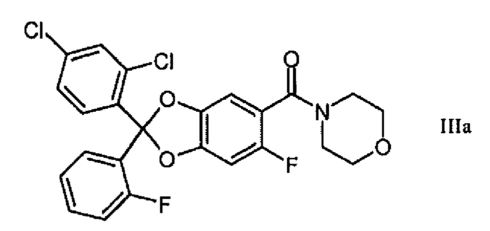 et l'isolement de l'isomère R de formule I par HPLC chirale préparative.

8. Composé de formule IIIa, qui est la 2-(2,4-dichloro-phényl)-2-(2-fluorophényl)-6-fluoro-benzo[1,3]dioxole-5-yl-morpholine-4-yl-méthanone.

9. Compositions pharmaceutiques comprenant un composé suivant l'une quelconque des revendications 1 à 5 et un support et/ou adjuvant pharmaceutiquement acceptable.

10. Composés suivant l'une quelconque des revendications 1 à 5, destinés à être utilisés comme substances thérapeutiques actives.

11. Composés suivant l'une quelconque des revendications 1 à 5, destinés à être utilisés comme substances thérapeutiques actives pour le traitement et/ou la prophylaxie de maladies qui sont associées à la modulation du récepteur CB1.

12. Utilisation de composés suivant l'une quelconque des revendications 1 à 5 pour la préparation de médicaments destinés au traitement et/ou la prophylaxie de maladies qui sont associées à la modulation des récepteurs CB1.
